# EUROPEAN PATENT APPLICATION

(11) **EP 3 100 668 A1**
(43) Date of publication of application: **07.12.2016**
(21) Application number: 14880937.9
(22) Date of filing: 30.10.2014
(51) Int. Cl.: A61B 1/04

(54) **MEDICAL VIDEO RECORDING AND PLAYBACK SYSTEM AND MEDICAL VIDEO RECORDING AND PLAYBACK DEVICE**

(30) Priority: 30.01.2014 JP 2014016101
(71) Applicant: Olympus Corporation, Tokyo 192-8507 (JP)
(72) Inventor: TSUCHIYA, Noriyoshi, Hachioji-shi, Tokyo 192-8507 (JP); SHINODA, Toru, Hachioji-shi, Tokyo 192-8507 (JP)
(74) Representative: Gunzelmann, Rainer
(86) International application number: PCT/JP2014/078975
(87) International publication number: WO 2015/114901

(57) **Abstract**

In order to provide a technique that provides high operability and makes it easy to find an insertion route to an affected area in an endoscopic examination, an image process unit 11 of a medical video recording and reproducing system 100 generates video data of a subject on the basis of a captured-image signal from an image capturing unit 3. A characteristic point information generation unit 14 generates, for a specified frame in video data, characteristic point information. A server 4 records characteristic point information and video data in an associated manner. A reproduction control unit 16 controls reproduction of a past video on the basis of at least one piece of video data read from the server 4. A synthesization unit 12 generates synthesized image data obtained by synthesizing live video data from the image process unit 11 and past video data from the reproduction control unit 16 in such a manner that the live video and the past video are displayed simultaneously, and outputs an obtained synthesized image data. The monitor 2 displays a synthesized image based on the synthesized image data. The reproduction control unit 16 conducts control of pausing reproduction at a frame to which the characteristic point information of the past video data is added.

## Description

### Technical Field

The present invention relates to a medical video recording and reproducing system and a medical video recording and reproducing device that record a medical video captured by using a medical device, and reproduce the recorded video or a video that is being captured.

### Background Art

Conventionally, in order to reconfirm an affected area found in previous endoscopic examinations, an image of the affected area is searched for from among endoscopic images captured in the past examinations so as to display the still image in a PinP (Picture in Picture) manner. Alternatively, an endoscopic image recorded by using a VTR is displayed in a PinP manner. The operator refers to a still image or a past endoscopic image of the affected area being displayed in a PinP manner, finds a location corresponding to the current examination, and makes a diagnosis etc. on the basis of changes in the affected area from the previous condition.

Regarding a medical image recording device capable of displaying and reproducing a beneficial video through a simple manipulation and in a short period of time, a technique of detecting a characteristic point from an obtained medical video so as to reproduce the video from the point in time of the detection of the characteristic point for reproduction of the video is disclosed (Patent Document 1, for example).

### [Prior art document]

### [Patent document]

[Patent document 1] Japanese Laid-open Patent Publication No. 2011-36370

### [Summary of the Invention]

### [Problem to be solved by the invention]

A keyboard etc. connected to an endoscopic device is used for conducting a manipulation of pausing a past image in a method in which an endoscopic examination is conducted while an image that was captured and obtained in the past is being reproduced and the reproduction is paused at a location showing an affected area so as to compare the area with a corresponding location in the image that is being captured. Because of this, the operator who is conducting an examination in the sterilized zone cannot conduct a manipulation of pausing the reproduction of past images by himself or herself, making it necessary for a person, other than the operator, in a non-sterilized zone to conduct this manipulation.

Also, according to the technique disclosed by Patent Document 1 above, reproduction starts from a location at which a characteristic point was detected on a past video. Because the image is not reproduced from the start of the examination, it is not easy for the operator to find the insertion route, sometimes making it troublesome to find a location of the affected area in an endoscopic examination.

As described above, there is a demand for a technique that provides high operability for an operator as a user of an endoscopic device and that makes it easy for such an operator to imagine an insertion route to an affected area in an endoscopic examination.

It is an object of the present invention to provide a technique that provides high operability and makes it easy to find an insertion route to an affected area in an endoscopic examination.

### [Solving means]

An aspect of the present invention is a medical video recording and reproducing system for recording and reproducing medical video data, the system including: an image capturing unit that obtains a captured-image signal by capturing an image of a subject; a video data generation unit that generates video data of the subject on the basis of the captured-image signal; a characteristic point information generation unit that generates, for a specified frame, characteristic point information representing an image having a character as an image of the subject from among frames that constitute the video data; a recording unit that records the characteristic point information and a corresponding frame of the video data in an associated manner; a reproduction control unit that controls reproduction of at least one past video on the basis of at least one piece of video data read from the recording unit; a synthesization unit that generates synthesized image data obtained by synthesizing live video data generated by the video data generation unit and past video data provided by the reproduction control unit in such a manner that a live video based on the live video data and a past video based on the past video data are displayed simultaneously, and that outputs an obtained synthesized image data; and a display unit that displays a synthesized image based on the synthesized image data output from the synthesization unit, wherein the reproduction control unit conducts control of pausing reproduction at a frame to which the characteristic point information of the past video data is added.

Another aspect of the present invention is a medical video recording and reproducing device that records and reproduces medial video data, including: an image capturing unit that obtains a captured-image signal by capturing an image of a subject; a video data generation unit that generates video data of the subject on the basis of the captured-image signal; a characteristic point information generation unit that generates, for a specified frame, characteristic point information representing an image having a character as an image of the subject from among frames that constitute the video data; a reproduction control unit that controls reproduction of at least one past video on the basis of at least one piece of video data read from a recording unit that records the characteristic point information and a corresponding frame of the video data in an associated manner; and a synthesization unit that generates synthesized image data obtained by synthesizing live video data generated by the video data generation unit and past video data provided by the reproduction control unit in such a manner that a live video based on the live video data and a past video based on the past video data are displayed simultaneously, and that outputs an obtained synthesized image data to a display unit, wherein the reproduction control unit conducts control of pausing reproduction at a frame to which the characteristic point information of the past video data is added.

### [Effect of the invention]

The present invention provides high operability and makes it easy for the operator to find an insertion route to an affected area in an endoscopic examination.

### [Brief Description of Drawings]

FIG. 1 shows the entire configuration of a medical video recording and reproducing system;
FIG. 2 is an overall block diagram of the medical video recording and reproducing system;
FIG. 3 exemplifies a synthesized image displayed on a monitor on the basis of synthesized image data obtained by synthesization in a synthesization unit;
FIG. 4 is a detailed block diagram showing the synthesization unit in a video processor of an endoscopic observation device;
FIGs. 5 exemplify windows for selecting a display mode;
FIG. 6 shows a specific example of a display mode; and
FIG. 7 shows an example of changing a display format on a monitor.

### [Mode of Carrying Out the Invention]

Hereinafter, the embodiments of the present invention will be explained in detail by referring to the drawings.

### <First embodiment>

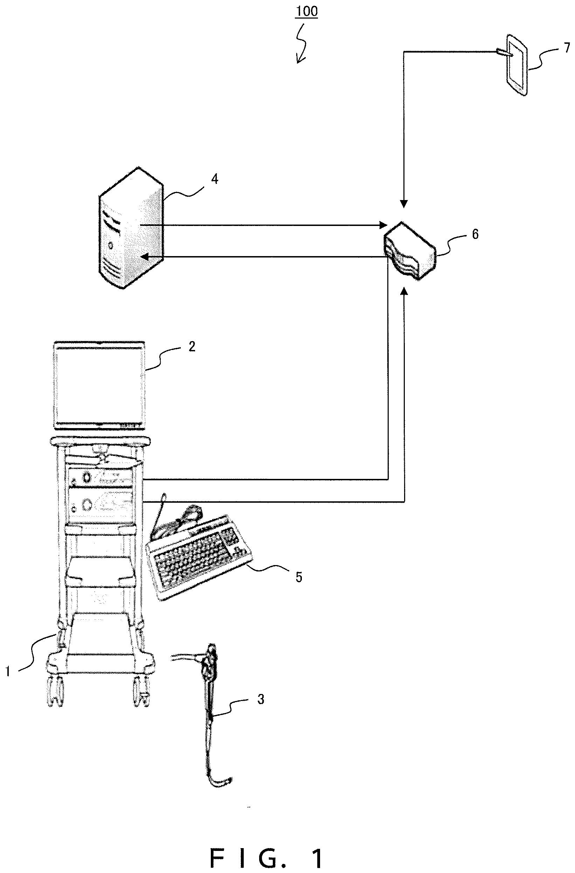

FIG. 1 shows the entire configuration of a medical video recording and reproducing system according to the present embodiment. A medical video recording and reproducing system 100 shown in FIG. 1 includes an endoscopic observation device 1, a monitor 2, scope 3, an image filing server (referred to as a server hereinafter) 4, a keyboard 5, a wireless LAN (local area network) router 6 and a tablet PC (personal computer) 7.

The medical video recording and reproducing system 100 shown in FIG. 1 uses the endoscopic observation device 1 to perform necessary processes on a captured-image signal obtained by the scope 3 in an endoscopic examination, and obtains video data. The obtained video data is recorded in the server 4. The video data recorded in the server 4 can also be read and processed by a video processor in the endoscopic observation device 1 so as to be reproduced by the monitor 2. Endoscopic examinations, the recording of video data, inputting or setting of various pieces of information necessary for reproduction can be conducted via various input units such as a manipulation switch of the scope 3, the keyboard 5, the tablet PC 7, etc.

Among various devices constituting the medical video recording and reproducing system 100, the endoscopic observation device 1 is a device in which devices such as a video processor, a light source device, the monitor 2, etc. are integrated in a trolley manner, and performs a necessary process on a medical video obtained by the scope 3 so as to make the monitor 2 output and display it.

The scope 3 has its tip inserted into the body cavity of the subject so as to output an obtained captured-image signal of the body cavity to the endoscopic observation device 1. The monitor 2 receives from the endoscopic observation device 1 video data resulting from performing a captured-image signal process by using the video processor of the endoscopic observation device 1, and displays the video on the screen.

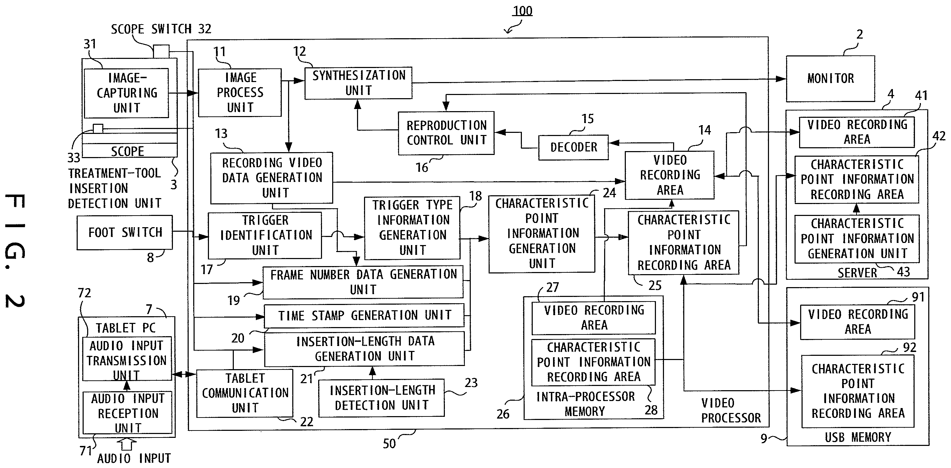

The server 4 receives via the wireless LAN router 6 video data obtained by performing an image process in the endoscopic observation device 1 so as to record the received video data. In the present embodiment, the server 4 records patient information for identifying a subject, i.e., a patient, with characteristic point information representing a frame image specified by the operator etc. from among pieces of video data, in an associated manner. Characteristic point information will be explained in detail later by referring to FIG. 2 or other figures.

As shown in FIG. 1, the present embodiment employs a configuration in which the endoscopic observation device 1, the server 4 and the tablet PC 7 are connected to each other via the wireless LAN, whereas the present invention is not limited to this example, and they may be connected via a wired LAN. Also, a network between the respective devices are not limited to a LAN, and various known networks may be for it.

The keyboard 5 is an example of input units for inputting various settings and instructions to the endoscopic observation device 1 to which it is connected. Examples of settings and instructions input by the keyboard 5 include instructions regarding reproduction of video data on the monitor 2 such as reproduction and pausing etc. of the video data and instructions such as addition etc. of characteristic point information in addition to setting and instructions related to an endoscopic examination.

Similarly to the keyboard 5, the tablet PC 7 is an example of input devices for inputting instructions regarding reproduction of video data on the monitor 2 and instructions such as addition etc. of characteristic point information.

In the medical video recording and reproducing system 100 according to the present embodiment, in an endoscopic examination, the endoscopic observation device 1 first confirms whether or not the server 4 has already recorded a piece of video data having the identical patient information on the basis of the patient information for identifying the patient who is going to received the examination. When the server 4 has already recorded a piece of video data having the identical patient information, the video data is transmitted to the endoscopic observation device 1 via the wireless LAN router 6. The endoscopic observation device 1 live displays a video received from the scope 3 on the monitor 2, and also displays a past video on the basis of video data received from the server 4 and stored in a memory etc. of the endoscopic observation device 1. On the monitor 2, a past video is reproduced and the live video that is being captured by the scope 3 is displayed. The operator inserts the scope 3 into the body cavity of the patient and determines a location that needs detailed checking, i.e., a location showing an affected area on the past video, by referring to the past video, and thereby conducts observation.

Manipulations of reproduction, pausing, fast-forwarding, fast-rewinding, etc. of a video can be conducted through a manipulation switch located at a position, in the scope 3, close to a hand of the operator, a foot switch (not shown) located close to a foot of the operator in FIG. 1, and the keyboard 5 manipulated by a staff member etc. in an non-sterilized zone. In addition to this, when for example reconfirmation of a video recorded in the server 4 is to be conducted after an examination, the tablet PC 7 etc. can be used for it. As described above, a manipulation of adding characteristic point information to video data can also be conducted by using these units.

According to the medical video recording and reproducing system 100 of the present embodiment, a frame image showing an affected area etc. and the above characteristic point information are recorded in an associated manner during an endoscopic examination. When an endoscopic examination is to be conducted later again, a past video is read from the server 4 and is reproduced and displayed on the monitor 2, and the reproduction of the past video is paused at a location of a frame image to which the characteristic point information has been added. Specific explanation will be given for a method in which the medical video recording and reproducing system 100 adds characteristic point information to video data so as to use it in future endoscopic examinations.

FIG. 2 is an overall block diagram of the medical video recording and reproducing system 100 according to the present embodiment. In FIG. 2, as constituents of the medical video recording and reproducing system 100, the keyboard 5 and the wireless LAN router 6 shown in FIG. 1 are omitted and a foot switch 8 and a USB (universal serial bus) memory 9 are added. FIG. 2 shows only constituents related to recoding and reproducing of a medical video according to the present embodiment, and other constituents are omitted.

The scope 3 includes an image-capturing unit 31, a scope switch 32 and a treatment-tool insertion detection unit 33. The image-capturing unit 31 has a lens and a CCD (charge coupled device), and obtains a captured-image signal of the subject. The scope switch 32 is provided to a manipulation unit that is used by the operator nearby his or her hand, so as to feed, to video processor 50 of the endoscopic observation device 1, various manipulations of endoscopic examinations and instructions of adding of a characteristic point information to a video that is being captured. A treatment-tool insertion detection unit 38 includes for example a sensor provided to a forceps hole provided to the tip of the scope 3, and the sensor detects that the operator as the user used forceps via the forceps hole. A method of utilizing a result of detection by the treatment-tool insertion detection unit 38 will be described in detail in the explanations for a first variation example.

The foot switch 8 is used for inputting a manipulation of the scope 3 during an endoscopic examination or a surgery and various manipulations related to reproduction of a video and adding of characteristic point information.

The tablet PC 7 includes an audio input reception unit 71 and an audio input transmission unit 72. In the present embodiment, when the audio input reception unit 71 receives an audio input from the user, the audio input transmission unit 72 transmits to the video processor 50 the audio data input to the tablet PC 7. The video processor 50 receives the audio data transmitted from the audio input transmission unit 72 of the tablet PC 7 via a tablet communication unit 22.

Note that the video processor 50 that has received audio data obtains necessary information by analyzing it in an analysis unit (not shown in FIG. 2), and feeds the obtained information to respective units that constitute the video processor 50. Communications between the tablet PC 7 and the video processor 50 are not limited to the audio data communication exemplified in FIG. 2.

The video processor 50 of the endoscopic observation device 1 includes an image process unit 11, a synthesization unit 12, a recording video data generation unit 13, a video recording area 14, a decoder 15, a reproduction control unit 16, a trigger identification unit 17, a trigger type information generation unit 18, a frame number data generation unit 19, a time stamp generation unit 20, an insertion-length data generation unit 21, the tablet communication unit 22, an insertion-length detection unit 23, a characteristic point information generation unit 24, a characteristic point information recording area 25 and an intra-processor memory 26. The video processor 50 performs a necessary process on a captured-image signal obtained through the capturing by using the image-capturing unit 31 of the scope 3, so as to obtain video data, and outputs the video data to the monitor 2.

In the video processor 50, the image process unit 11 performs a necessary image process on the captured-image signal input from the scope 3 so as to obtain video data. The synthesization unit 12 generates synthesized image data by synthesizing video data input from the image process unit 11, i.e., the live video data, with past video data read from the server 4 or the USB memory 9 in such a manner that a live video based on the live video data and a past video based on the past video data are displayed simultaneously on the monitor 2, and transmits it to the monitor 2.

The recording video data generation unit 13 includes for example an encoder, and performs a necessary process such as encoding etc. on video data input from the image process unit 13. In recording video data, a frame number, a time stamp, and various pieces of information such as the insertion-length of the scope 3 etc. may be recorded in an associated manner in addition to the above characteristic point information.

The video recording area 14 is an area for temporarily storing recording video data generated by the recording video data generation unit 13 or video data read from the server 4 or the USB memory 9.

The decoder 15 decodes video data held by the video recording area 14. Decoded video data is fed to the reproduction control unit 16.

The reproduction control unit 16 controls reproduction of a past video on the basis of the video data resulting from the decoding by the decoder 15 and the characteristic point information associated with that video data. The reproduction control unit 16 outputs, to the synthesization unit 12 together with the characteristic point information, the past video data for which the reproduction is controlled.

Explanations will be given for constituents, of the video processor 50, related to the generation of characteristic point information.

The trigger identification unit 17 recognizes a manipulation by a user that functions as a trigger for associating characteristic point information with the frame information corresponding to the information. A "manipulation that functions as a trigger" refers to a manipulation on the scope switch 32, the foot switch 8, the tablet PC 7, the keyboard 5 (shown in FIG. 1 but not shown in FIG. 2), etc. Communications with the tablet PC 7 are conducted via the tablet communication unit 22 as described above.

The trigger type information generation unit 18 generates information that represents which of the units were used for inputting the user's manipulation that was recognized by the trigger identification unit 17. Specific examples of trigger type information include the scope 3 (and the scope switch 32 and the treatment-tool insertion detection unit 33 of the scope 3), the foot switch 8, the tablet PC 7, the keyboard 5, etc.

When a manipulation of adding characteristic point information is conducted by the scope 3 etc., the frame number data generation unit 19 generates data representing the frame number of a corresponding frame image. The time stamp generation unit 20 detects the time and date at which the manipulation of adding the characteristic point information was conducted, and generates a time stamp. On the basis of the insertion length of the scope 3 at the timing of the manipulation of adding the characteristic point information detected by the insertion-length detection unit 23, the insertion-length data generation unit 21 generates insertion length data of the scope 3. In the insertion-length data generation unit 21, a known technique is used for generating insertion length data from the detection result by the insertion-length detection unit 23. A specific method of utilizing insertion length data will be described in detail in the explanations for a second variation example.

The characteristic point information generation unit 24 generates characteristic point information from data input from the trigger type information generation unit 18, the frame number data generation unit 19, the time stamp generation unit 20 and the insertion-length data generation unit 21. Characteristic point information includes the frame number of a frame image, the time stamp, the insertion length of the scope 3 corresponding to the timing at which the user of the medical video recording and reproducing system 100 such as the operator or other people manipulated the scope switch 32 etc. Characteristic point information may include other pieces of information or may include some of the above pieces of information. A frame image to which the characteristic point information has been added indicates that it is a frame, specified by the user on the video, showing an affected area etc.

The characteristic point information recording area 25 is an area for holding characteristic point information generated in the video processor 50. In an embodiment, in addition to characteristic point information, a shooting condition such as an observation mode (normal light observation mode, narrow-band light observation mode, fluorescence observation mode, infrared light observation mode, etc.) etc. are stored in the characteristic point information recording area 25. The video processor 50 stores in the server 4 and the USB memory 9 information such as characteristic point information etc. of the characteristic point information recording area 25 in a state that it is associated with video data held by the video recording area 14.

Returning to the explanations of operations of the reproduction control unit 16, the reproduction control unit 16 refers to the frame number of characteristic point information and pauses the reproduction at the frame having the frame number corresponding to the characteristic point information on a past video.

On the basis of the live video data input from the image process unit 11 and past video data input from the reproduction control unit 16, the synthesization unit 12 generates synthesized image data to be displayed on the monitor 2, and outputs the generated synthesized image data to the monitor 2. When a past video is paused by the reproduction control unit 16, synthesized image data of a state in which the past video became still at a frame with characteristic point information is generated, and is output to the monitor 2. The monitor 2 displays the synthesized image on the basis of the synthesized image received from the synthesization unit 12.

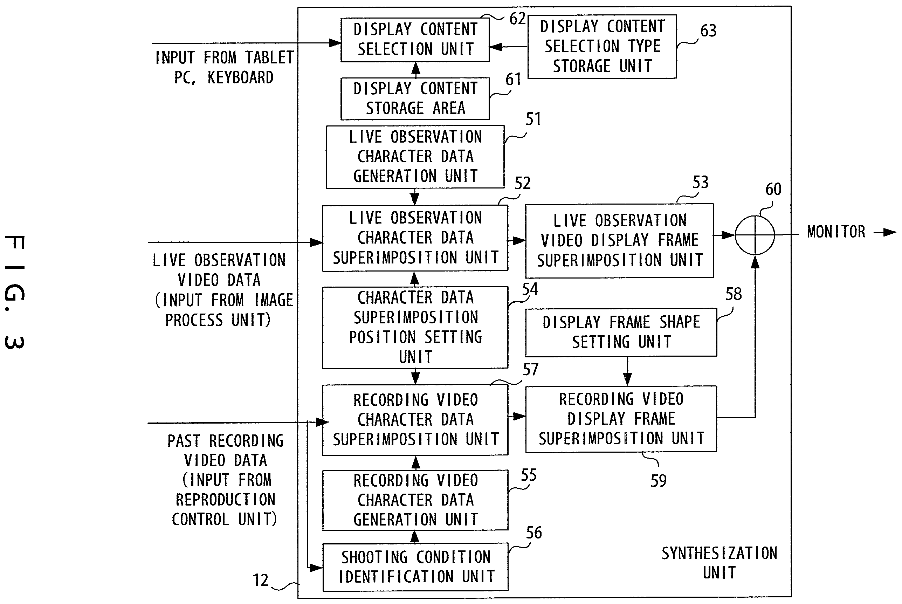

FIG. 3 exemplifies a synthesized image displayed on the monitor 2 on the basis of synthesized image data obtained by the synthesization in the synthesization unit 12.

In the example shown in FIG. 3, live video P1 and past video P2 are displayed side by side on the monitor 2. It is also possible to display progress bar PB at a position close to an edge of past video P2 as shown in FIG. 3.

Progress bar PB in FIG. 3 shows a relative position of a timing at which characteristic point information is added on a video in the period between the start and end of the video of a past endoscopic examination and also shows a period of time that has elapsed from the start of the video. Specifically, shooting time T of past video P2 is 20 minutes, and pieces of characteristic point information f1, f2 and f3 have been added to video P2. The fact that pieces of characteristic point information f1, f2 and f3 have been added at the timings of "5 minutes", "7 minutes" and "12 minutes" from the start of the video is displayed so that the operator etc. as the user can recognize it easily and visually.

The operator refers to progress bar PB so as to start an examination by inserting the scope 3 (start of the capturing of a video), and thereafter recognizes a location to which characteristic point information is added, i.e., a period of time that it will take for the scope 3 to reach the affected area etc. As described above, past video P2 is paused at frames to which pieces of characteristic point information f1 through f3 have been added. The operator refers to progress bar PB so as to determine the position of the affected area etc. , compares the live video and past frame images of affected area etc. to which characteristic point information f1 through f3 have been added, and conducts an examination.

As described above, the video processor 50 shown in FIG. 2 records, in the server 4 and the USB memory 9, past video data to be synthesized with a live video by the synthesization unit 12 in a state that it is associated with characteristic point information.

The server 4 has a video recording area 41, a characteristic point information recording area 42 and a characteristic point information generation unit 43. The video recording area 41 is an area for recording video data received from the video processor 50 of the endoscopic observation device 1. The characteristic point information recording area 42 is an area for recoding characteristic point information received from the video processor 50 together with video data. Operations of the characteristic point information generation unit 43 are similar to those of the characteristic point information generation unit 24 of the video processor 50, and generates characteristic point information from information input directly to the server 4 or input indirectly via a network.

The USB memory 9 has a video recording area 91 and a characteristic point information recording area 92. Similarly to the video recording area 41 and the characteristic point information recording area 42 of the server 4, the video recording area 91 and the characteristic point information recording area 92 are areas respectively for holding video data and characteristic point information.

Note that the intra-processor memory 26 is a storage unit provided in the video processor 50. As shown in FIG. 2, the intra-processor memory 26 has a video recording area 27 and a characteristic point information recording area 28. Similarly to the server 4 and the USB memory 9, the video recording area 27 and the characteristic point information recording area 28 are areas respectively for holding video data and characteristic point information.

As described above, according to the medical video recording and reproducing system 100 of the present embodiment, in the video processor 50 of the endoscopic observation device 1, a video of a past examination of the same patient (information) is displayed on the monitor 2 together with the live video, i.e., the image that is being captured in an endoscopic examination. A past video is reproduced from the start of the examination, and is paused at a point to which characteristic point information is added. This makes it possible for the operator to find easily an insertion route of the scope 3 to the affected area so as to conduct an examination without the necessity of asking a staff member in a non-sterilized zone to pause the video at a location of the affected area etc. to which characteristic point information was added in a past examination.

Note that the above explanations are based on a case where a live video and a past video are both displayed side by side on one monitor 2, whereas the method of recording and reproducing a medical video according to the present embodiment is not limited to that example. For example, a live video and a past video may be displayed on separate monitors. This makes it possible for the user to determine arbitrarily the arrangements of the monitors in accordance with the use conditions etc. of the monitors 2.

### <First variation example>

In the above, characteristic point information is added via a manipulation switch etc. of the scope 3, the keyboard 5 connected to the endoscopic observation device 1, or the tablet PC 7 that can conduct wireless communications via the wireless LAN router 6. By contrast, in the present variation example, the treatment-tool insertion detection unit 33 shown in FIG. 2 recognizes the insertion of a treatment tool such as forceps etc. , and this is determined to be a trigger for adding characteristic point information.

The video processor 50 of the endoscopic observation device 1, detecting insertion of a treatment tool such as forceps etc. via the forceps hole of the scope 3, determines that there is an affected area etc. at the position of the scope 3 at the time of the detection of the insertion. Then, characteristic point information is added to the frame image at the time of the detection of the insertion of forceps.

This configuration makes it easy to associate characteristic point information to a frame image, on video data, that should be referred to in a reexamination etc.

### <Second variation example>

In the above configuration, a start of an endoscopic examination activates the reproduction of a past video. By contrast, in the present variation example, a past video is reproduced from a location corresponding to the insertion length of the scope 3 specified by the user of the medical video recording and reproducing system 100 by using an input unit such as the keyboard 5, the tablet PC 7, etc.

As described above, characteristic point information includes the insertion length of the scope 3. Utilizing this, the video processor 50 calculates the frame number corresponding to an insertion length specified by the user from a plurality of pieces of insertion length data of characteristic point information, and the video is reproduced from the location having that frame number.

Thereby, in a case when a long time from the start of an examination is taken to reach the frame to which the first characteristic point information is added or when the fact that only an image of a prescribed location is necessary is known beforehand, or in other cases, it is possible to reproduce a video from a location at which the user needs the reproduction to start.

### <Second embodiment>

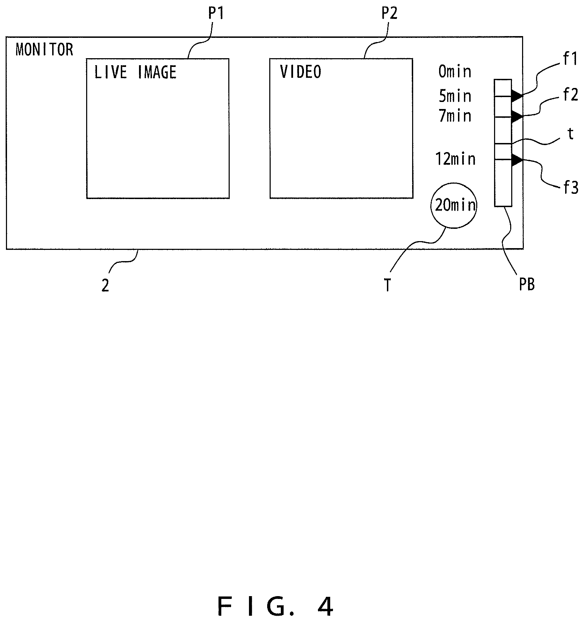

In the above embodiment, the live video and a past video are displayed side by side on the monitor 2 as shown in FIG. 3. The medical video recording and reproducing system 100 of the present embodiment employs a method of displaying images on the monitor 2 that has taken into consideration convenience of the user such as the operator etc. who conducts an examination while comparing the live video and a past video. Hereinafter, by referring to FIG. 4 through FIG. 7, specific explanations will be given for a method of displaying videos on the monitor 2, employed by the medical video recording and reproducing system 100 of the present embodiment.

Hereinafter, the explanations will be focused on aspects different from those of the above embodiment, and aspects similar to those of the above embodiment will not be explained. The configuration of the medical video recording and reproducing system 100 is as explained above by referring to FIG. 1 or FIG. 2.

FIG. 4 is a detailed block diagram showing the synthesization unit 12 in the video processor 50 of the endoscopic observation device 1. By referring to FIG. 4, specific explanations will be given for how to generate a synthesized image to be displayed on the monitor 2 after synthesizing a live video and a past video.

As shown in FIG. 4, the synthesization unit 12 includes a live observation character data generation unit 51, a live observation character data superimposition unit 52, a live observation video display frame superimposition unit 53, a character data superimposition position setting unit 54, a recording video character data generation unit 55, a shooting condition identification unit 56, a recording video character data superimposition unit 57, a display frame shape setting unit 58, a recording video display frame superimposition unit 59, an addition unit 60, a display content storage area 61, a display content selection unit 62 and a display content selection type storage area 63.

When a live video and a past video are displayed on the monitor 2 respectively on the basis of live video data input from the image process unit 11 shown in FIG. 2 and past video data input from the reproduction control unit 16, the character data superimposition position setting unit 54 sets the position of a character etc. that represents whether each video is a live video or a past video. The position of a character etc. is determined on the basis of the content set by the user via the tablet PC 7, the keyboard 5, etc. shown in FIG. 1.

The live observation character data generation unit 51 generates character data indicating that the corresponding video is a live video from among two video types displayed on the monitor 2. The live observation character data superimposition unit 52 superimposes character data generated by the live observation character data generation unit 51 on live video image input from the image process unit 11. The live observation video display frame superimposition unit 53 further superimposes a display frame for a live video on data resulting from superimposing the character data on the live video in the live observation character data superimposition unit 52.

The shooting condition identification unit 56 obtains a shooting condition of a video from information input from the reproduction control unit 16. Examples of shooting conditions include an observation mode, i.e., whether the shooting is conducted as normal light observation or a special light observation (narrow-band light observation, fluorescence observation, infrared light observation, etc.). The recording video character data generation unit 55 generates character data indicating that the corresponding video is a past video and representing its shooting condition on the basis of the shooting condition obtained by the shooting condition identification unit 56, from among the two videos displayed on the monitor 2. The recording video character data superimposition unit 57 superimposes the character data generated by the recording video character data generation unit 55 on past video data input from the reproduction control unit 16. The recording video display frame superimposition unit 59 further superimposes a display frame for past frame on data resulting from superimposing the character data on the past video in the recording video character data superimposition unit 57.

In the present embodiment, as a display frame for a past video, a shape etc. that is different from a display frame for a live video is set so that the user can easily distinguish it from a live video on the monitor 2. The display frame shape setting unit 58 sets a display frame for a past video in accordance with the display mode set by the user via an input unit such as the tablet PC 7, a keyboard, etc. The recording video display frame superimposition unit 59 superimposes, on the past video and the character data, a display frame for a past video set in the display frame shape setting unit 58.

In the addition unit 60, pieces of data obtained by superimposing character data and a display frame respectively on a live video and a past video are disposed at prescribed positions on the windows so as to generate synthesized image data to be displayed on the monitor 2, and the data is output to the monitor 2.

As described above, in the medical video recording and reproducing system 100 of the present embodiment, it is possible for the user to select a desired character or a display frame regarding what is used as a character and a display frame to be superimposed and regarding how to use them. The display content selection unit 62 receives content selected by the user for a character and a display frame, via the above various input unit such as the tablet PC 7 etc. The display content selection type storage area 63 is an area for storing the type of various characters and display frames that can be expressed and that can be provided by the medical video recording and reproducing system 100. The display content storage area 63 is an area for storing types of various characters and display frames, selected by the user, that are stored in the display content selection type storage area 63. Hereinafter, the type of a display format such as a character, display frame, etc. that the medical video recording and reproducing system 100 can provide to the user is referred to as a "display mode".

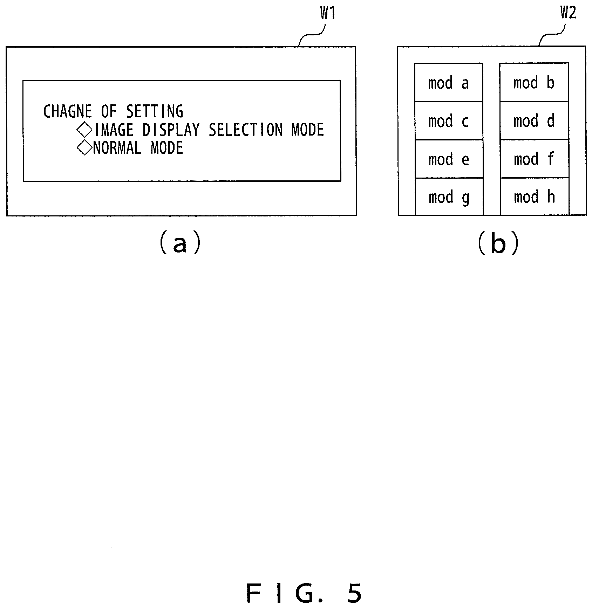

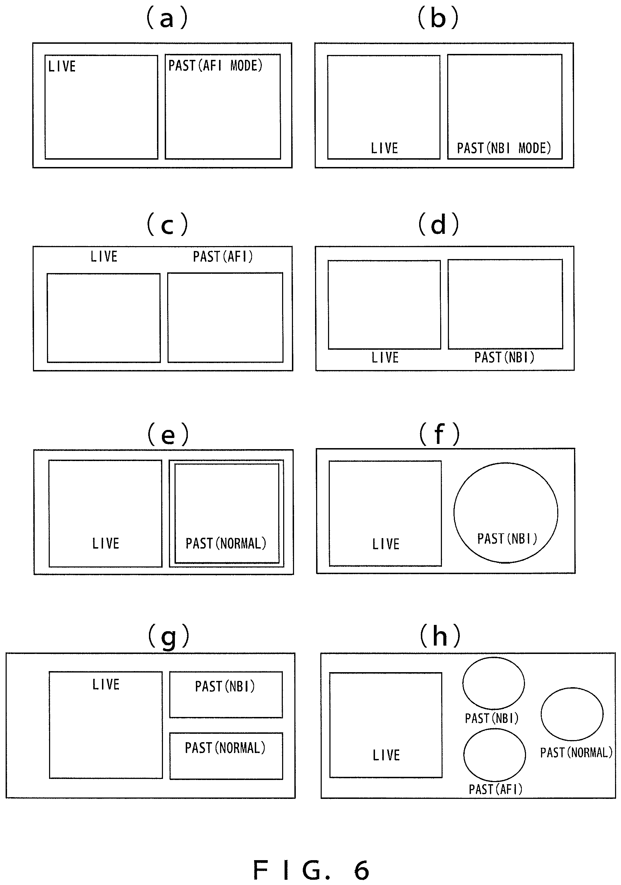

In the present embodiment, the user can select a display mode via a mode selection window displayed on the monitor 2. This will be explained by referring to FIG. 5 and FIG. 6.

FIGs. 5 exemplify selectin windows of a display mode. FIG. 5(a) exemplifies display mode setting changing window W1, and FIG. 5 (b) exemplifies window W2 that displays a list of display modes that the user can set.

When the user selects "image display selection mode" via the tablet PC 7, a keyboard, etc. in display mode setting changing window W1 shown in FIG. 5(a), the video processor 50 of the endoscopic observation device 1 displays display mode list window W2 of FIG. 5 (b) on the monitor 2 in accordance with information stored in the display content storage area 61. When the user selects "normal mode" in display mode setting changing window W1 shown in FIG. 5(a), the video processor 50 switches the display to for example the window exemplified in FIG. 3 in response to the display content selection type storage area 63 starting to hold information of a character and a display frame displayed on the monitor 2 in the normal mode.

Eight display modes are exemplified in display mode list window W2 shown in FIG. 5(b). The user selects one of the display modes in display mode list window W2 shown in FIG. 5 (b) . The synthesization unit 12 makes the display content selection type storage area 63 hold information of a character and a display frame corresponding to the selected display mode, and thereafter, a video is displayed on the monitor 2 in accordance with thus held information.

Note that window W2 may display a sample window of each display mode so that the mode is set to the mode selected by the user, although this is omitted in FIG. 5(b) due to space limitations. Specific examples of display modes are shown in FIG. 6(a) through FIG. 6(h).

In the display modes shown in FIG. 6 (a) and FIG. 6(b), characters "LIVE" and "PAST (AFI mode)", which respectively represent a live video and a past video, are displayed in the display frames of the respective videos. In FIG. 6(a), the characters are displayed in an upper portion in the display frame, and in FIG. 6 (b) the characters are displayed in a lower portion in the display frame. As shown, the present embodiment displays characters indicating that the video is a past video, together with characters indicating that the observation mode of the past video is the AFI (fluorescence observation) mode.

In the display modes shown in FIG. 6 (c) and FIG. 6(d), characters indicating that the videos are respectively a live video and a past video are displayed out of the display frame of each video. In FIG. 6 (c), the characters are displayed in an upper portion that is out of and close to the display frame, and in FIG. 6(d), the characters are displayed in a lower portion that is out of and close to the display frame.

The display modes in FIG. 6 (e) and FIG. 6 (f) display the display frames for past videos in a color and shape different from those for live videos so that the user will not confuse the live video and the past video on the monitor 2. The display frame of a past video is enclosed by double rectangles in FIG. 6(e) and the display frame of a past video is circular in FIG. 6 (f) so that they have shapes different from the single-rectangular display frame for the live video.

In the display modes shown in FIG. 6 (g) and FIG. 6(h), a plurality of past videos are displayed simultaneously on the monitor 2. In FIG. 6(g), two past videos are displayed together with the live video and the past videos are for the purpose of references during the current examination and accordingly the past videos are displayed in a size relatively smaller than that of the live video. Also, the present embodiment also displays a shooting condition of each past video so that the user can recognize relationships between past videos. FIG. 6(h) shows a case where three past videos are displayed together with the live video. Similarly to FIG. 6(g), the past videos are displayed in a size smaller than that of the live video. The display frames of the past videos are circular so that the user can recognize them at a glance. Similarly to FIG. 6(g), the shooting condition of each of the past videos is also displayed.

Note that display modes are not limited to those exemplified in FIG. 6, and these display modes may be combined on an as-needed basis.

Also, FIG. 6 exemplifies a case where a character is used for representing an observation mode as the shooting condition of a past video, whereas a shooting mode represented by a character is not limited to this example. In addition to an observation mode or in place of an observation mode, information such as for example information for identifying a scope, information of enlargement magnification, etc. maybe displayed. It is also possible to employ a configuration in which the user selects whether or not these shooting conditions are to be displayed and, in case of displaying shooting condition, whether or not which piece of such shooting condition information is to be displayed so that displaying is conducted in accordance with the selection.

Also, in addition to making a setting change via a display mode changing window as exemplified by FIGs. 5, it is also possible to employ a configuration in which the user can change the setting via a manipulation etc. on a touch panel monitor or the tablet PC 7 in order to move the position a character displayed on a window from the default position. Thereby, the display mode is changed from for example a mode having a character displayed in an upper portion in the display frame as shown in FIG. 6 (a) to a display mode having a character displayed in a portion that is out of and close to the display frame as shown in FIG. 6 (c).

Regarding a shooting condition displayed in a form of a character, information recorded in association with video data is read from the server 4, the USB memory 9 or the video processor 50 and the read information is displayed.

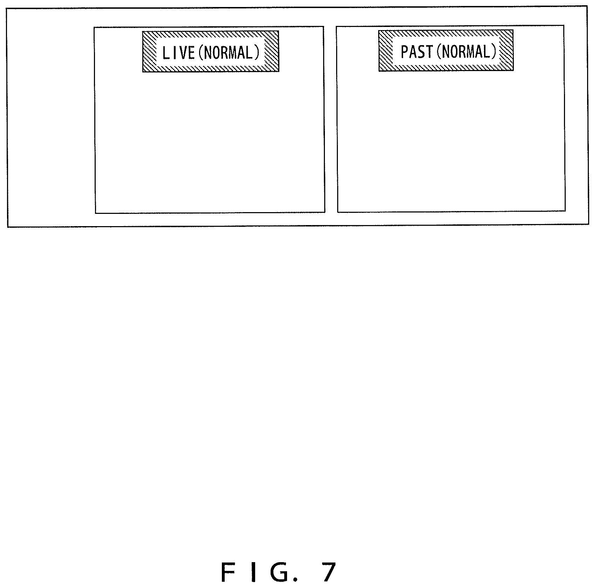

It is further possible to employ a configuration in which the synthesization unit 12 of the video processor 50 changes a display format in addition to the state of a video. This will be explained by referring to FIG. 7.

FIG. 7 explains an example of changing a display format on the monitor 2.

Endoscopic observation images are dark in some cases, depending upon observation modes or observation positions in the body cavities. If an image is dark in a display mode of displaying various characters in the display frames, it is difficult to read characters in such display frames. In view of above, the colors of characters may be changed to for example a while color by comparing the value of the color of the shaded area in FIG. 7 with a prescribed threshold so as to determine that the value of the color in the shaded area has become closer to the value of the color of the characters. As described above, the colors of characters are changed automatically in real time so that the contrast between the colors of characters and the background portion becomes sharper, and thereby a situation is effectively prevented in which it is difficult for the user to read characters in some images.

It is also possible to employ a configuration in which the user can arbitrarily select whether or not to change the character colors. In other words, when the user selects a setting of fixed character colors, it is possible to prevent effectively a situation etc. where the colors of characters displayed on the monitor 2 change frequently in accordance with the values of colors in the image.

As explained above, according to the medical video recording and reproducing system 100 of the present embodiment, the user can set a desired display mode so that the user such as the operator can easily know which of a plurality of videos being displayed on the monitor 2 is the live image during an endoscopic procedure. By displaying the live video and a past video in accordance with this, the user can arrange respective videos at desired positions, making it possible to prevent effectively a situation where the user confuses the live videos and past videos.

Also, the present invention is not limited to the above embodiments as they are, but can be embodied by modifying the constituents in the practical phases without departing from the spirit of the invention. Also, various inventions can be formed by an appropriate combination of the plurality of constituents disclosed in the above embodiments. For example, all the constituents disclosed in the above embodiments may be combined appropriately. Further, constituents may be combined appropriately across different embodiments. As a matter of course, these various modifications and applications are possible without departing from the spirit of the invention.

### [Explanations of numerals]

- 1: endoscopic observation device
- 2: monitor
- 3: scope
- 4: image filing server (server)
- 5: keyboard
- 6: wireless LAN router
- 7: table PC
- 50: video processor
- 100: medical video recording and reproducing system

## Claims

1. A medical video recording and reproducing system for recording and reproducing medical video data, the system comprising:
an image capturing unit that obtains a captured-image signal by capturing an image of a subject;
a video data generation unit that generates video data of the subject on the basis of the captured-image signal;
a characteristic point information generation unit that generates, for a specified frame, characteristic point information representing an image having a character as an image of the subject from among frames that constitute the video data;
a recording unit that records the characteristic point information and a corresponding frame of the video data in an associated manner;
a reproduction control unit that controls reproduction of at least one past video on the basis of at least one piece of video data read from the recording unit;
a synthesization unit that generates synthesized image data obtained by synthesizing live video data generated by the video data generation unit and past video data provided by the reproduction control unit in such a manner that a live video based on the live video data and a past video based on the past video data are displayed simultaneously, and that outputs an obtained synthesized image data; and
a display unit that displays a synthesized image based on the synthesized image data output from the synthesization unit, wherein
the reproduction control unit conducts control of pausing reproduction at a frame to which the characteristic point information of the past video data is added.

2. The medical video recording and reproducing system according to claim 1, wherein
the synthesization unit generates the synthesized image in which a progress bar representing a reproduction state of a past video that has been reproduced after being read from the recording unit is further synthesized, and outputs the synthesized image to the display unit.

3. The medical video recording and reproducing system according to claim 1 or 2, wherein
the synthesization unit generates the synthesized image on which a character is superimposed, the character indicating that a video being captured and a past video that are displayed on the display unit are respectively a video being captured and a past video, and outputs the synthesized image to the display unit.

4. The medical video recording and reproducing system according to claim 3, wherein
the synthesization unit generates, for each of a video being captured and a past video that are displayed on the display unit, the synthesized image on which a specified display frame is further superimposed, and outputs the synthesized image to the display unit.

5. The medical video recording and reproducing system according to claim 4, wherein
the synthesization unit generates the synthesized image on which a character representing an observation mode of the past video to be displayed is further superimposed, and outputs the synthesized image to the display unit.

6. The medical video recording and reproducing system according to claim 5, wherein
the display unit displays types of a character and a display frame that can be selected by a user and that are superimposed on the video, and the synthesization unit generates the synthesized image by using a character and a display frame selected by a user from among the types displayed on the display unit.

7. The medical video recording and reproducing system according to claim 6, wherein
the characteristic point information generation unit generates the characteristic point information at a timing specified via a manipulation switch of an endoscopic device, a foot switch of the endoscopic device, a keyboard of the medical video recording and reproducing system and a terminal device connected to the medical video recording and reproducing system.

8. The medical video recording and reproducing system according to claim 6, wherein
the characteristic point information generation unit generates the characteristic point information at a timing at which insertion of forceps through a forceps hole of an endoscopic device is detected.

9. The medical video recording and reproducing system according to claim 6, further comprising
an insertion length detection unit that detects an insertion length of an insertion unit of an endoscopic device into a body cavity of the subject, wherein
the reproduction control unit reproduces the past video from a location corresponding to an insertion length of the insertion unit specified by a user.

10. A medical video recording and reproducing device that records and reproduces medial video data, the device comprising:
an image capturing unit that obtains a captured-image signal by capturing an image of a subject;
a video data generation unit that generates video data of the subject on the basis of the captured-image signal;
a characteristic point information generation unit that generates, for a specified frame, characteristic point information representing an image having a character as an image of the subject from among frames that constitute the video data;
a reproduction control unit that controls reproduction of at least one past video on the basis of at least one piece of video data read from a recording unit that records the characteristic point information and a corresponding frame of the video data in an associated manner; and
a synthesization unit that generates synthesized image data obtained by synthesizing live video data generated by the video data generation unit and past video data provided by the reproduction control unit in such a manner that a live video based on the live video data and a past video based on the past video data are displayed simultaneously, and that outputs an obtained synthesized image data to a display unit, wherein
the reproduction control unit conducts control of pausing reproduction at a frame to which the characteristic point information of the past video data is added.
